# EUROPEAN PATENT APPLICATION

(11) **EP 4 438 030 A1**
(43) Date of publication of application: **02.10.2024**
(21) Application number: 24382294.7
(22) Date of filing: 18.03.2024
(51) Int. Cl.: A61K 8/11, A61K 8/34, A61K 8/63, A61K 8/67, A61K 8/9789, A61Q 19/02

(54) **TERNARY ANTI-SPOT COMBINATION TO LIGHTEN SKIN**

(30) Priority: 21.03.2023 ES 202330233
(71) Applicant: E Grau Active Cosmetics, S.L.U., 08029 Barcelona (ES)
(72) Inventor: GRAU MONJO, Elena, 08029 BARCELONA (ES); ANTÓN RODRÍGUEZ, Laura, 08029 BARCELONA (ES)
(74) Representative: Sugrañes, S.L.P.

(57) **Abstract**

The present invention relates to a ternary anti-spot combination to lighten skin comprising hydroxytyrosol, masilinic acid and vitamin C. It also relates to nanocapsules comprising said combination. The present invention also relates to the use of said combination and said nanocapsules for the dermocosmetic treatment of the skin, and to a dermocosmetic composition comprising said nanocapsules.

## Description

### TECHNICAL SECTOR

The present invention relates to a ternary combination to lighten skin and to remove dark spots and marks from same.

### STATE OF THE ART

Skin spots are the result of the different distribution of melanin and the cells producing same, i.e., melanocytes, a type of cells in the epidermis. Specifically, melanogenesis takes place within melanosomes, which are vesicles within melanocytes.

Melanin is a dark pigment responsible for skin colour that offers protection against the sun's rays; therefore, after exposure to the sun, melanocytes secrete more melanin.

Melanogenesis is a complex process that is regulated by a series of multistep signal transduction cascades and influenced by a variety of extrinsic and intrinsic factors. As a result of this complex process, melanin synthesis can be activated by different stimuli, for example, solar radiation, inflammation, acne, stress, hormonal changes, pollution or inflammatory processes, among others.

The melanin generated in melanocytes is transferred to neighbouring keratinocytes, so the pigment becomes visible on the surface of the epidermis and progressively darkens due to photooxidation.

Different types of spots can be found on the skin. For example, freckles, moles, melanoma, lentigos, or melasma.

Freckles are brownish, rounded and non-uniform accumulations of pigment that are more frequently located in photo-exposed areas such as the face, neck and arms in fair-skinned people, intensifying with exposure to the sun, therefore being more visible in summer.

Moles are accumulations of a greater number of melanocytes affecting the different structures of the skin.

Melanoma is a malignant skin tumour that can be confused with a mole. It looks like a pigmented spot, with asymmetrical growth, imprecise-irregular borders and intensely pigmented colouring with areas that are less pigmented or even bluish in tone. It has progressive growth.

Simple and solar lentigines are small brownish spots that appear anywhere on the body and, unlike freckles, do not change colour with exposure to the sun.

Melasma are light or dark brown spots that appear almost exclusively in women and are caused or aggravated by pregnancy (chloasma), taking contraceptives or menopause.

While melanin plays a key role in protecting the skin from harmful ultraviolet (UV) radiation, abnormally high melanin production and accumulation in the skin can lead to hyperpigmentation. Although usually harmless, hyperpigmentation of the skin, especially on the face, is generally considered unsightly. Therefore, concern about the appearance of the skin has prompted research into cosmetic products for whitening skin, which are capable of reducing skin pigmentation. These products are generally used to remove different types of pigment spots on the skin or to lighten a naturally dark skin colour or to prevent skin pigmentation.

The mechanisms involved in skin pigmentation and prevention skin care agents are described, for example, in Kumari et al., Melanogenesis inhibitors, Acta Derm. Venereol., 2018, 98, 924-931.

In the state of the art, different approaches have been described to obtain skin depigmentation or skin lightening. These include reducing melanin production by inhibiting tyrosinase, inhibiting the transfer of mature melanosomes by melanin containing keratinocytes, or neutralising reactive oxygen species (ROS) in the skin (which activate melanogenesis) and reducing direct photo-oxidation of pre-existing melanin by means of using antioxidants.

The paper by L. Azcona, Problemas de pigmentación. Tratamiento, Farmacia Profesional, 2003, 17(1), 72-76, describes the main active agents used in treatments for cutaneous depigmentation. These include hydroquinone, rucinol (4-n-butyl resorcinol), arbutin (glucose derivative of hydroquinone), azelaic acid, kojic acid, phytic acid, N-acetyl-4-S-cysteaminophenol, Sohakuhi extract (*Morus nigra*), licorice extract (*Glycyrrhiza gabra*), aloe extract (*Aloe barbadensis*), sorrel extract (*Rumex occidentalis*), pilosella extract (*Hieracium pilosela*), yarrow extract (*Achillea millefolium*), as tyrosinase inhibitors. It also describes lipoic acid, which inhibits non-enzymatic melanin synthesis and degrades enzymatic melanin, and retinoic acid (tretinoin), which decreases melanogenesis in active melanocytes. Antioxidants such as ascorbic acid and its derivatives, such as magnesium L-ascorbyl phosphate, reduce intermediate compounds in the synthesis of melanin, and they also inhibit tyrosinase.

Bernard et al., Resveratrol: an original mechanism on tyrosinase inhibition, Int. J. Cosmet. Sci., 2000, 22(3), 219-226, describe that polyphenols, such as resveratrol, have skin lightening effects through tyrosinase inhibition; and Spanish patent application ES-A-2462565 describes the dermocosmetic use of hydroxytyrosol to inhibit the synthesis of melanin and reduce skin colouring or to lighten, whiten and/or produce depigmentation of human skin.

Some substances to lighten skin, such as, for example, soy milk and soy extracts, and niacinamide, are believed to inhibit the transfer of mature melanosomes by melanin which contains keratinocytes, as described in Hakozaki et al., The effect of niacinamide on reducing cutaneous pigmentation and suppression of melanosome transfer, Br. J. Dermatol., 20-31, and in Paine et al., An Alternative Approach to Depigmentation by Soybean Extracts via Inhibition of the PAR-2 Pathway, J. Invest. Dermatol., 2001, 116(4), 587-595.

Another approach to removing skin spots is based on the use of α- and β-hydroxy acids, such as glycolic acid, salicylic acid, or pyruvic acid as depigmenting agents as a result of a peeling effect.

Skin lightening active ingredients can also be in encapsulated form. For example, international patent application WO-A-88/09659 describes liposomes comprising a combination of hydroquinone and kojic acid, and international patent application WO-A-2021/052647 describes a skin lightening composition comprising sclareolide, kojic acid, and ascorbyl glucoside, wherein kojic acid is encapsulated within a micro- or nanocapsule comprising a specific peptide.

Skin treatments are generally by topical application of creams or lotions, but Chinese patent application CN-A-106511108 describes a mask comprising resveratrol, proanthocyanidins, and hydroxytyrosol as skin lightening active ingredients.

Compositions comprising peptides in combination with other skin lightening active ingredients have also been described, for example in European patent applications EP-A-3988080 and EP-A-3928760.

Despite the diversity of active ingredients and compositions for removing skin spots and lightening the skin described up to now in the state of the art, there is still a need for safer compositions, free of undesirable adverse and toxic effects, which, in particular, have a strong anti-melanogenic effect, and thus achieve an improved effect with smaller amounts of active ingredients and in a shorter period of time.

### SUMMARY OF THE INVENTION

The present invention relates to a ternary anti-spot combination to lighten skin.

In another aspect, the invention relates to nanocapsules comprising said combination.

In another aspect, the invention relates to a dermocosmetic composition comprising said nanocapsules.

In another aspect, the invention relates to the use of said combination and of said nanocapsules to prepare a dermocosmetic composition for the treatment of human skin.

### FIGURES

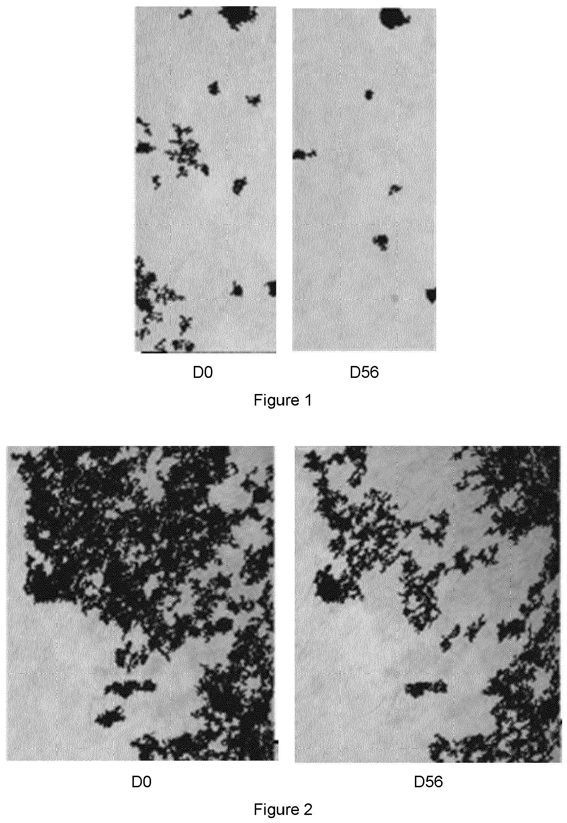

Figure 1 shows an image of the skin obtained with a Bio3D scanner combined with a multispectral imaging system at the beginning of the trial (D0) and another image after 56 days (D56) of treatment with a dermocosmetic cream as described in Example 6. The reduction in skin pigmentation as a result of treatment with said cream can be clearly observed.
Figure 2 shows an image of the skin obtained with a Bio3D scanner combined with a multispectral imaging system at the beginning of the trial (D0) and another image after 56 days (D56) of treatment with a dermocosmetic cream as described in Example 6. The reduction of the area of the spots as a result of treatment with said cream can be clearly observed.

### DETAILED DESCRIPTION OF THE INVENTION

The present invention relates to a ternary anti-spot combination to lighten human skin comprising:
a) hydroxytyrosol,
b) maslinic acid and
c) vitamin C.

In one embodiment, the ternary anti-spot combination essentially consists of hydroxytyrosol, maslinic acid and vitamin C.

In one embodiment, the ternary anti-spot combination consists of hydroxytyrosol, maslinic acid and vitamin C.

The authors of the present invention have developed a ternary anti-spot combination to lighten skin that allows to reduce skin colouring, lighten, whiten and/or produce depigmentation of human skin. Although the melanin reduction process is a complex and, above all, very slow process, surprisingly, said combination achieves statistically significant results with respect to the reduction of skin spots, to the increase in skin luminosity, to the reduction of pigmentation and the improvement in skin colour uniformity in a short period of time of 7 days. These results are observed in a magnified manner after the application of said combination over 14, 28 and 56 days.
In the present description, as well as in the claims, the singular forms "a" and "the" include plural reference unless the context clearly indicates otherwise.

In this description, all percentages (%) are expressed by weight (w/w), unless indicated otherwise, and in the compositions, the sum of the percentages of the different components is adjusted to add up to 100% in each case.

Numerical values preceded by the term "about" or "around" are intended to also include a certain variation around said value, namely, a variation of ±5% from the indicated amount. The numerical intervals defined by lower and upper end points, using the expressions "between ... and ..." and "from ... to ...", also include said indicated end points and also include any narrower sub-interval.

In the context of the invention, a cosmetic active agent or cosmetic active ingredient is any substance intended to be applied to the body surface, particularly on the skin, hair or nails, to provide a cosmetic effect. A cosmetic effect refers to beautifying and/or improving the feel or sensory aspects of normal, non-diseased skin, hair or nails. Cosmetic effects do not imply any therapeutic effect, in other words, cosmetics are not intended to prevent or improve any disease. A cosmetic effect is, for example, skin whitening.

In the context of the invention, the terms "whitening", "lightening" or "depigmentation" referring to human skin, are used interchangeably herein and refer to the process of lightening hyperpigmented skin, typically, to remove or reduce dark spots, defects or marks on the skin, including age spots, post-acne marks, uneven skin tone, post-inflammatory hyperpigmentation spots or melasma and lentigo, for example.

In the context of the invention, a dermocosmetic composition is aimed at skin care, the purpose of which is to treat certain non-pathological conditions of human skin, in order to improve its dermatological appearance. Typically, a dermocosmetic composition is somewhere between the category of cosmetics and medicinal products, functioning as effective complements to medical treatments and as enhancers of some aspects of the field of beauty. Therefore, a dermocosmetic composition generally provides an effective method to prevent and combat skin problems, such as spots. In general, the user establishes a long-term care routine so that the dermocosmetic composition has the desired effect on the skin.

In the present description, the terms "spots", "marks" and "blemishes" are equivalent and used interchangeably.

### Anti-spot ternary combination

In one aspect, the invention relates to a ternary anti-spot combination to lighten human skin comprising hydroxytyrosol, maslinic acid and vitamin C.

### Hydroxytyrosol

Hydroxytyrosol is a phenolic compound that comes from the olive tree (*Olea europaea*), particularly from the fruit and its leaves, and is considered a powerful antioxidant. The chemical name of hydroxytyrosol is 2-(3,4-dihydroxyphenyl)ethanol and the accession number in the Chemical Abstracts database is 10597-60-1.

In the combination of the invention, hydroxytyrosol is in pure form or in the form of an *Olea europaea* extract.

The pure product, generally with a purity of 98% or greater as determined by HPLC, is commercially available through, for example, the company Sigma-Aldrich.

*Olea europaea* extracts with a hydroxytyrosol content of at least 40% (w/w) are also commercially available, for example, through the company egactive cosmetics.

In a preferred embodiment, hydroxytyrosol is in the form of an *Olea europaea* extract, the extract preferably comprising at least 40% (w/w) hydroxytyrosol.

### Maslinic acid

Maslinic acid is a triterpene compound that belongs to the oleanane group and comes from the olive tree (*Olea europaea*), particularly the fruit and leaves thereof. The chemical name of maslinic acid is (4a*S*,6a*S,*6b*R*,8a*R*,10*R,*11*R,* 12a*R*,12b*R*,14b*S*)-10,11-dihydroxy-
2,2,6a,6b,9,9,12a-heptamethyl-1,3,4,5,6,6a,6b,7,8,8a,9,10,11,12,12a,12b,13,14b-octadecahydropicene-4a(2*H*)-carboxylic acid, and the accession number in the Chemical Abstracts database is 4373-41-5.

In the combination of the invention, maslinic acid is in pure form or in the form of an *Olea europaea* extract.

The pure product, generally with a purity equal to or greater than 98%, determined by HPLC, is commercially available through, for example, the company Sigma-Aldrich.

*Olea europaea* extracts with a maslinic acid content of at least 60% (w/w) are also commercially available, for example, through the company egactive cosmetics.

In a preferred embodiment, maslinic acid is incorporated into the combination of the invention in the form of an *Olea europaea* extract, the extract preferably comprising at least 60% (w/w) maslinic acid.

### Vitamin C

Vitamin C is the chemical compound referred to as ascorbic acid or L-ascorbic acid, the accession number of which in the Chemical Abstracts database is 50-81-7. It is a water-soluble vitamin found in citrus fruits and in other fruits and vegetables.

In the context of the invention, the term "vitamin C" refers to both vitamin C, in other words, ascorbic acid, and to any of its derivatives, in particular, ascorbyl palmitate, sodium ascorbate, 3-O-ethyl L-ascorbic acid tetraisopalmitate ascorbate, ascorbyl glucoside, magnesium ascorbyl phosphate, and sodium ascorbyl phosphate.

Vitamin C can be incorporated into the combination of the invention as such, in other words, as ascorbic acid, or as one of its derivatives. The appropriate derivatives to be used in said combination include, for example, ascorbyl palmitate (6-O-palmitoyl L-ascorbic acid, CAS no. 137-66-6), sodium ascorbate (L-sodium ascorbate, CAS no. 134-03-2), tetraisopalmitate ascorbate (tetrahexyldecyl ascorbate, CAS no. 183476-82-6), 3-O-ethyl L-ascorbic acid (CAS no. 86404-04-8), ascorbyl glucoside (2-O-a-D-glucopyranosyl L-ascorbic acid, CAS no. 129499-78-1), magnesium ascorbyl phosphate (L-ascorbic acid 2-phosphate sesquimagnesium salt hydrate, CAS No. 1713265-25-8), and sodium ascorbyl phosphate (L-ascorbic acid 2-phosphate trisodium salt, CAS no. 66170-10-3).

In the combination of the invention, vitamin C selected from ascorbic acid, ascorbyl palmitate, sodium ascorbate, tetrahexyldecyl ascorbate, 3-O-ethyl ascorbic acid, ascorbyl glucoside, magnesium ascorbyl phosphate, sodium ascorbyl phosphate, and mixtures thereof, is used; preferably ascorbyl palmitate is used.

Vitamin C and its derivatives are commercially available, for example, through the companies Sigma-Aldrich or Essentials of Australia.

In the ternary combination, hydroxytyrosol is generally comprised in a percentage between 20% (w/w) and 60% (w/w), preferably between 25% (w/w) and 55% (w/w), more preferably between 30% (w/w) and 50% (w/w), and even more preferably between 39% (w/w) and 43% (w/w); maslinic acid is generally comprised in a percentage between 12% (w/w) and 45% (w/w), preferably between 18% (w/w) and 40% (w/w), more preferably between 20% (w/w) and 34% (w/w), and even more preferably between 25% (w/w) and 29% (w/w); and vitamin C is generally comprised in a percentage between 15% (w/w) and 50% (w/w), preferably between 20% (w/w) and 45% (w/w), more preferably between 25% (w/w) and 40% (w/w), and even more preferably between 30% (w/w) and 35% (w/w), expressed as ascorbic acid equivalent; wherein all the percentages with respect to the total weight of the ternary combination and the sum of the percentages of the three components is 100%.

### Nanocapsules

In one aspect, the invention relates to nanocapsules comprising:
a) the ternary anti-spot combination to lighten human skin,
b) a biodegradable polymer, and
c) at least one cosmetically acceptable ingredient.

### Biodegradable polymer

A biodegradable polymer is generally a derivative of renewable materials which has benefits with respect to conventional polymers mainly derived from petroleum, such as, for example, polyethylene, polypropylene, PVC, polystyrene, polycarbonate and polymethyl methacrylate. Said biodegradable polymer is characterised by having a non-toxic nature, biocompatibility, degradability, sustainability, and extreme hydrophilicity, as described in, for example, Alaswad et al., Recent Advances in Biodegradable Polymers and Their Biological Applications: A Brief Review, Polymers, 2022, 14, 4924.

In the scope of the invention, biodegradable polymer is selected, for example, from polylactic acid (PLA), polyglycolic acid (PGA), poly(lactic-co-glycolic acid) (PLGA), albumin, polycaprolactone (PCL), polybutyrate adipate terephthalate (PBAT), polyesteramides (PEA), polyhydroxyvalerate (PHV), polyalkylene alkanoates (PBS), and thermoplastic starch (TPS); preferably from polylactic acid (PLA), polyglycolic acid (PGL), poly(lactic-co-glycolic acid) (PLGA), albumin, polycaprolactone (PCL), and polyesteramides (PEA); more preferably from polylactic acid (PLA), polyglycolic acid (PGL), poly(lactic-co-glycolic acid) (PLGA), and polycaprolactone (PCL); and even more preferably the biodegradable polymer is polycaprolactone.

Biodegradable polymers are commercially available from various companies, such as, for example, BASF, Perstorp, Corbion, or Chemours.

### Cosmetically acceptable ingredient

In the context of the invention, cosmetically acceptable means that the compositions, formulations or components described are suitable for use in contact with human skin without undue toxicity, incompatibility, instability, allergic response and the like.

The nanocapsules comprise at least one cosmetically acceptable ingredient. In general, said ingredient is selected from surfactants, emulsifiers, lipid compounds, emollients, consistency factors, thickeners, stabilisers, hydrotropes, preservatives, antioxidants, essences, colourants, silicone compounds, fats, waxes, lecithins, phospholipids, film-forming agents, and mixtures thereof; preferably selected from surfactants, emulsifiers, lipid compounds (e.g. vegetable oil, triglycerides, diglycerides, monoglycerides), film-forming agents, and mixtures thereof. In a more preferred embodiment, the cosmetically acceptable ingredient is the combination of sorbitan stearate, polyvinyl alcohol, and triglycerides of capric and caprylic acids.

Examples of cosmetically acceptable ingredients are cited in particular in the International Cosmetic Ingredient Dictionary and Handbook published by the Cosmetic, Toiletry, and Fragrance Association (CTFA). Cosmetically acceptable ingredients, as well as their characteristics, can also be found in Rowe et al., Ed., Handbook of Pharmaceutical Excipients, Pharmaceutical Press, London, 2003.

In one embodiment, the nanocapsules are in solid form, preferably in powder form. In general, the average size (D50) of the nanocapsules is between 400 and 700 nm, preferably between 500 and 600 nm, with a polydispersity index of about 0.5. The particle size distribution can be determined by the dynamic light scattering technique, which allows determining the particle size between 1 nm and 10 microns, for example, with Zetasizer equipment from the company Malvern.

Nanocapsules generally comprise between 5% (w/w) and 20% (w/w), preferably between 7% (w/w) and 18% (w/w), more preferably between 10% (w/w) and 15% (w/w), of the ternary combination; between 50% (w/w) and 70% (w/w), preferably between 52% (w/w) and 68% (w/w), more preferably between 55% (w/w) and 65% (w/w), of a biodegradable polymer; and between 15% (w/w) and 35% (w/w), preferably between 20% (w/w) and 30% (w/w), more preferably between 23% (w/w) and 28% (w/w), of a cosmetically acceptable component. The percentages refer to the total weight of the nanocapsules, and component percentages add up to 100% (w/w).

In another embodiment, the nanocapsules are in the form of a dispersion in an aqueous carrier, preferably water. In general, the dispersion comprises the nanocapsules in solid form at a concentration comprised between 0.05% (w/w) and 2% (w/w), preferably between 0.1% (w/w) and 2% (w/w), more preferably between 0.15% (w/w) and 0.5% (w/w), and more preferably around 0.2% (w/w).

The dispersion of the capsules generally comprises a cosmetically acceptable ingredient that is selected from surfactants, emulsifiers, thickeners, sequestrants, antioxidants, stabilisers, hydrotropes, preservatives, cosolvents, anti-foaming agents, and mixtures thereof.

In a preferred embodiment, the cosmetically acceptable ingredient is selected from thickeners, antioxidants, preservatives, and mixtures thereof.

In a more preferred embodiment the cosmetically acceptable ingredient comprises a thickener, an antioxidant, and one or more preservatives.

The thickeners that can be used include xanthan gum, carbomer, ethyl cellulose, hydroxyethyl cellulose, hydroxypropyl cellulose, hydroxypropyl methylcellulose, carboxymethyl cellulose, carraghenate, chitosan, guar gum, and mixtures thereof. In a preferred embodiment, the thickener is xanthan gum. The thickener content in the dispersion is generally comprised between 0.1% (w/w) and 5% (w/w), preferably between 0.2% (w/w) and 2% (w/w), and more preferably between 0.4% (w/w) and 1% (w/w).

The antioxidants that can be used include tocopherol, butylated hydroxyanisole (BHA), butylated hydroxytoluene (BHT), ascorbic acid, sodium ascorbate, ascorbyl palmitate, sodium bisulphite, sodium metabisulphite, sodium sulphite, phytic acid, sodium phytate, and mixtures thereof. In a preferred embodiment, the antioxidant is sodium phytate. The antioxidant content in the dispersion is generally between 0.01% (w/w) and 0.5% (w/w), preferably between 0.02% (w/w) and 2% (w/w), and more preferably between 0.05% (w/w) and 0.1% (w/w).

The preservatives that can be used include benzalkonium chloride, benzoic acid, sodium benzoate, potassium benzoate, benzyl alcohol, butylparaben, propylparaben, methylparaben, chlorohexidine, sorbic acid, potassium sorbate, propionic acid, potassium propionate, phenoxyethanol, and mixtures thereof. In a preferred embodiment, the preservative is selected from potassium sorbate, sodium benzoate, and a mixture thereof. The preservative content, or mixture of preservatives, in the dispersion is generally comprised between 0.1% (w/w) and 5% (w/w), preferably between 0.2% (w/w) and 2% (w/w), and more preferably between 0.4% (w/w) and 1.5% (w/w).

Typically, the nanocapsule dispersion is used in dermocosmetic compositions at a dose comprised between 0.5% (w/w) and 5% (w/w), preferably between 1% (w/w) and 2% (w/w), with respect to the total weight of the dermocosmetic composition.

### Method to prepare the nanocapsules

The nanocapsules can be prepared according to methods described in the state of the art, such as, for example, in Spanish patent application EP-A-0349428, or in Diyanat et al., Preparation and characterization of polycaprolactone nanocapsules containing pretilachlor as a herbicide nanocarrier, Envir. Sci. Poll. Res., 2019, 26, 21579-21588.

The method generally comprises:
1) adding an organic phase to an aqueous phase under stirring, wherein the organic phase comprises a biodegradable polymer, a lipid compound, a surfactant, a film-forming agent, the ternary combination, and an organic solvent, and the aqueous phase comprises water, optionally a surfactant,
2) removing the solvent under reduced pressure.

The surfactant in the organic phase is typically a surfactant with a low hydrophilic-lipophilic balance (HLB), preferably comprised between 3 and 6, such as, for example, sorbitan stearate.

The surfactant in the aqueous phase is typically a surfactant with a high HLB, preferably comprised between 8 and 18, such as, for example, ethoxylated sorbitan monooleate with 20 moles of ethylene oxide.

Examples of surfactants with different HLB values can be found in, for example, X. Domingo, A guide to the surfactants world, Edicions Proa, Barcelona, 1995, and in Rowe *et al., op. cit.*

The organic solvent is generally selected from methanol, ethanol, isopropanol, acetone, and mixtures thereof.

### Dermocosmetic composition

One aspect of the invention relates to a dermocosmetic composition comprising the nanocapsules of the invention.

The dermocosmetic composition comprises:
a) nanocapsules, comprising the ternary combination of hydroxytyrosol, maslinic acid and vitamin C, and
b) a cosmetically acceptable component.

In one embodiment, the dermocosmetic composition contains at least one cosmetically acceptable component which, in the context of the invention, is selected from a cosmetically acceptable carrier, an additional cosmetic active component, an additional cosmetic ingredient, and mixtures thereof.

A cosmetically acceptable carrier (or vehicle) is a carrier in which cosmetic ingredients are dissolved, emulsified, dispersed or suspended. Said carrier is selected from water, a non-aqueous carrier miscible in water, such as, for example, ethanol or isopropanol, and a non-aqueous carrier not miscible in water, such as vegetable oil, fatty esters, medium chain triglycerides, or alkanes. Preferably, the dermocosmetic composition includes water as a carrier.

In one embodiment, the additional cosmetic active ingredient can be selected, for example, from skin soothing and healing agents, skin anti-aging agents, skin moisturising agents, anti-wrinkle agents, anti-fibrinolytic agents, anti-atrophy agents, skin softening agents, antibacterial agents, antifungal agents, antimicrobial agents, anti-inflammatory agents, antipruritic agents, external anesthetic agents, keratolytic agents, free radical scavengers, antiseborrheic agents, anti-dandruff agents, agents that modulate differentiation, proliferation or pigmentation of the skin and agents that accelerate penetration, agents that stimulate the synthesis of dermal or epidermal macromolecules and/or prevent their degradation, agents that stimulate the proliferation of fibroblasts and/or keratinocytes or stimulate the differentiation of keratinocytes, muscle relaxers, anti-pollution and/or anti-radical agents, slimming agents, anti-cellulite agents, agents that act on microcirculation, agents that act on the energy metabolism of cells, sunblocks and/or sunscreen compounds, makeup agents, antiseptic agents, deodorant active ingredients, cosmetic astringents, anti-acne agents, anti-caking agents, enzymes, enzyme inhibitors, enzyme inducing agents, coenzymes, plant extracts, plant tissue extracts, plant seed extracts, vitamins, vegetable oils, ceramides, peptides, cosmetic biocides, denaturants, astringent compounds, film formers, vitamins and derivatives thereof, exfoliating agents, lignans, UV absorbents, water soluble sunscreen, antiperspirant, antioxidants, hair remover, fat soluble sunscreen, skin restructuring agent, antiallergics, anti-irritants, immune system stimulants, immune system suppressants, insect repellents, lubricants, photostabilisers, and mixtures thereof.

The additional cosmetic ingredient is selected, for example, from surfactants, humectants, emulsifiers, lipid compounds, emollients, consistency factors, thickeners, stabilisers, hydrotropes, cosolvents, sequestrants, buffers, preservatives, perfumes, colourants, silicones, fats, waxes, lecithins, phospholipids, UV sunscreen factors, film-forming agents, self-tanners, firmers, pigments, colourants, acids and bases to adjust pH, and mixtures thereof.

In a preferred embodiment, the cosmetic composition contains an additional cosmetic ingredient selected from the group consisting of surfactants, humectants, cosolvents, emulsifiers, lipids, emollient compounds, consistency factors, thickening agents, silicones, perfumes, hydrotropes, preservatives, and mixtures thereof.

The dermocosmetic composition is preferably applied to the skin topically.

The dermocosmetic composition can be presented in any form typically used for topical application. Said composition can be presented in the form of a wide variety of product types including, among others, solid and liquid compositions such as aqueous solutions, hydroalcoholic solutions, oil solutions, lotions, creams, gels, bars, soaps, aerosols, ointments, pastes, powders, foams, mousses, milks, balsams, pomades, oil-in-water emulsions, water-in-oil emulsions, multiple emulsions (water/oil/water or oil/water/oil), microemulsions, nanoemulsions, dispersions, suspensions, or wipes. Said presentation forms and the methods for preparing them are well known to those skilled in the art and described, for example, in C. Fauli, Tratado de Farmacia Galénica, Luzan 5, Madrid, 1993, or in Remington, The Science and Practice of Pharmacy, 20th Ed., Lippincott Williams & Wilkins, Philadelphia, 2000.

The dermocosmetic composition of the present invention can be formulated in the form of a solution. The solutions may preferably include an aqueous solvent, for example, between about 75% (w/w) and about 98% (w/w) or between about 75% (w/w) and about 85% (w/w) of a cosmetically acceptable aqueous solvent. Said solvent may include, in addition to water, ethanol, propylene glycol, polyethylene glycol, mixtures thereof and the like. In one embodiment, the topical composition useful in the present invention can be formulated as a solution containing an emollient. Such a composition preferably contains between about 2% (w/w) and about 50% (w/w) of an emollient. As used herein, "emollients" refers to materials used for the prevention or relief of dryness, as well as for skin protection.

A lotion can be prepared from a solution. Lotions typically contain between about 1% (w/w) and about 20% (w/w) of an emollient and between about 50% (w/w) and about 90% (w/w) of water.

Another product type can be a cream. A cream typically comprises between about 5% (w/w) and about 50% (w/w) of an emollient and between about 45% (w/w) and about 95% (w/w) of water.

Another product type can be an ointment. An ointment can be made up of a simple base of vegetable oils or semi-solid hydrocarbons. An ointment may contain between about 2% (w/w) and about 100% (w/w) of an emollient, and between about 0.1% (w/w) and about 5% (w/w) of a thickening agent.

The dermocosmetic composition of the present invention can also preferably be formulated as an emulsion. In one embodiment, the cosmetic composition contains water and a lipophilic phase and is presented in the form of an emulsion or dispersion, such as oil in water (O/W), water in oil (W/O type), multiple emulsion (W/O/W or O/W/O), or a PIT type emulsion, or microemulsion. If the carrier is an emulsion, between about 1% (w/w) and about 10% (w/w) of the carrier should be made up of one or more emulsifiers. Emulsifiers can be non-ionic, anionic, cationic or amphoteric.

The dermocosmetic composition of the present invention can also be formulated as a gel. Suitable gelling agents for aqueous gels include, among others, natural gums, polymers and copolymers of acrylic acid and acrylate, and cellulose derivatives (for example, hydroxymethyl cellulose and hydroxypropyl cellulose). Gelling agents suitable for oils (such as vegetable oils, esters) include, among others, waxes, modified silicas, cellulose derivatives, polyamides, polyurethanes and L-glutamic acid derivatives. Such gels typically comprise between about 0.1% (w/w) and about 20% (w/w) of said gelling agents.

The dermocosmetic composition of the present invention can also be formulated in solid form, such as, for example, a wax-based bar, a mascara, a soap bar composition, a powder, or a wipe.

The dermocosmetic composition of the invention comprises between 0.5% (w/w) and 5% (w/w), preferably between 1% (w/w) and 2% (w/w), with respect to the total weight of the dermocosmetic composition, of the nanocapsule dispersion comprising the nanocapsules in solid form at a concentration between 0.05% (w/w) and 2% (w/w), preferably between 0.1% (w/w) and 2% (w/w), more preferably between 0.15% (w/w) and 0.5% (w/w), and more preferably around 0.2% (w/w).

### Dermocosmetic use of the combination

In another aspect, the invention relates to the use of said ternary combination and of said nanocapsules to prepare a dermocosmetic composition to lighten human skin.

In one embodiment, the dermocosmetic composition is used to reduce the formation of melanin and/or the oxidation thereof.

In one embodiment, the dermocosmetic composition is used to reduce the area of skin spots, reduce the contrast of dark spots against the skin, reduce skin pigmentation, the number of skin spots, increase skin luminosity, or increase skin uniformity.

One aspect of the invention relates to said ternary combination and said nanocapsules for use in dermocosmetic treatment to lighten human skin.

One aspect of the invention relates to a method to lighten human skin comprising the use of said ternary combination and said nanocapsules.

In one embodiment, the dermocosmetic composition is applied topically to the skin, preferably the skin of the face and hands.

The frequency of application of the dermocosmetic composition of the invention is generally 1 to 3 times, preferably 2 times, a day for a period of between 1 and 12 weeks, preferably between 1 and 8 weeks.

The effect of the ternary combination on skin lightening can be determined by measuring skin pigmentation (ITA), the area of dark spots, the number of dark spots, the contrast of dark spots against the skin, and skin uniformity, for example, by means of a colorimeter, as described, for example, in Ly et al., Research Techniques Made Simple: Cutaneous Colorimetry: A Reliable Technique for Objective Skin Color Measurement, J. Invest. Dermatol., 2020, 140(1), 3-12, or by means of a Bio3D scanner combined with a multispectral imaging system, as described in Mullor et al., Bio blue light scanner para evaluación de eficacia despigmentante, anti-manchas y anti-ojeras, npc, 2019, XLVIII (368), 4-9.Blue Light.

This effect can also be determined by means of a clinical evaluation *in vivo* of people who receive the application of the dermocosmetic composition of the invention. For example, expert assessments can be made at different times after the first application to evaluate skin luminosity and dark spots. To determine skin luminosity, a classification scale can be defined with photographs of the skin with different degrees of luminosity, where grade 1 corresponded to absence of luminosity, and grade 5 to very shiny skin. Likewise, a classification scale can be defined to determine dark spots, which comprised photographs of skin without dark spots (grade 1), up to grade 5, which corresponded to many dark spots.

The ternary combination has an effect on melanin production in melanocytes irradiated with ultraviolet light. Thus, as described in the examples, in the control group of melanocytes irradiated with ultraviolet light, without treatment with the ternary combination, melanin production increases by 31%, while a 49% reduction occurs in the group of melanocytes treated with the ternary combination of the invention, which is comparable to the 42% obtained in the melanocyte group treated with hydroquinone.

Therefore, the ternary combination of the invention surprisingly reduces the formation of melanin with an efficacy comparable to that of hydroquinone. This compound is used as a positive control, since it is an active ingredient indicated for the gradual lightening of skin spots, always under medical or pharmaceutical prescription.

Taking into account that the melanin reduction process is a complex and, above all, very slow process, it is surprising that the results obtained by means of treating only 7 days with a cream containing 2% (w/w) of the nanocapsule dispersion, such as that prepared in Example 2, are statistically significant with respect to the reduction of skin spots, to the increase in skin luminosity, to the reduction of pigmentation and the improvement in the uniformity of skin colour.

Thus, treatment for only 7 days with such cream leads to a reduction of the area of the spot by about 10% and the contrast of dark spots against the skin by about 8%. The results assessed over longer periods of time are also conclusive as to the skin lightening efficacy of the ternary combination of the invention, in particular, there are increases in the ITA value, indicating a decrease in skin pigmentation, in skin luminosity, and in skin uniformity, as well as a reduction in the area of dark spots, in the number of dark skin spots, and in the contrast of dark spots vs. skin.

As for skin compatibility and acceptability, none of the volunteers showed any skin acceptability problems or manifested a cutaneous reaction during treatment.

Studies carried out on human skin using nanocapsules of the invention loaded with a fluorescent marker show that these nanocapsules are capable of penetrating the basal layers of the epidermis, and thereby reaching the target cells.

Several examples are provided below by way of illustration, but not limitation of the invention.

### EXAMPLES

### Example 1: Capsules of the ternary anti-spot powder combination

The method for preparing polycaprolactone nanocapsules was adapted from the method described in Diyanat et al., Preparation and characterization of polycaprolactone nanocapsules containing pretilachlor as a herbicide nanocarrier, Envir. Sci. Poll. Res., 2019, 26, 21579-21588.

Nanocapsules of the anti-spot combination were prepared, which nanocapsules comprised 60% (w/w) of polycaprolactone as an encapsulating agent, about 10% (w/w) of ascorbyl palmitate (equivalent to about 4.25% (w/w) of ascorbic acid), 6.0% (w/w) of an *Olea europaea* extract containing 60% (w/w) of maslinic acid (corresponding to 3.6% (w/w) of maslinic acid), 14% (w/w) of an *Olea europaea* extract containing 40% (w/w) of hydroxytyrosol (corresponding to 5.6% (w/w) of hydroxytyrosol), 5% (w/w) of polyvinyl alcohol, 2.5% (w/w) of medium chain triglycerides, and 2.5 % (w/w) of sorbitan stearate.

At the end of the method, a powdery composition formed by nanocapsules that comprised the anti-spot combination of the invention was obtained. The mean size (D50) of the nanocapsules was between 400 and 700 nm, with a polydispersity index of about 0.5. The particle size distribution was determined with Zetasizer equipment from the company Malvern.

In the 12-month stability test carried out both at room temperature (22°C-25°C) and at 40°C, it was found that the size of the capsules remained substantially unchanged.

The recommended dose of this powder composition is comprised between 0.0002% (w/w) and 0.0004% (w/w).

### Example 2: Dispersion with capsules of the ternary anti-spot combination

The nanocapsules in solid form obtained in Example 1 were dispersed, under stirring at room temperature, in an aqueous solution containing water, xanthan gum as a thickener, sodium phytate as an antioxidant, and sodium benzoate and potassium sorbate as preservatives.

The dispersion obtained comprised 0.2% (w/w) of the nanocapsules in solid form of Example 1, 98.385% (w/w) of water, 0.5% (w/w) of xanthan gum, 0.085% (w/w) of sodium phytate, 0.45% (w/w) of sodium benzoate and 0.38% (w/w) of potassium sorbate.

In the 12-month stability test carried out both at room temperature (22°C-25°C) and at 40°C, it was found that the size of the capsules remained substantially unchanged.

The recommended dose of this dispersion in a dermocosmetic composition is comprised between 0.5% (w/w) and 5% (w/w), preferably between 1% (w/w) and 2% (w/w)

### Example 3: Dermocosmetic cream with nanocapsules of the anti-spot ternary combination

A dermocosmetic cream with the nanocapsule dispersion of Example 2 has the composition of Table I:

**TABLE I**

| Component | % (w/w) |
|---|---|
| Water | 72.95 |
| Propanediol | 1.50 |
| Glycerin | 1.50 |
| Sodium phytate | 0.10 |
| Niacinamide | 4.00 |
| Allantoin | 0.50 |
| Carbomer | 0.30 |
| Tranexamic acid | 2.00 |
| Pentylene glycol | 3.50 |
| Polyglyceryl-3-methylglucose distearate | 2.50 |
| Cetyl alcohol | 2.50 |
| Dimethicone | 3.50 |
| Dispersion, Example 2 | 5.00 |
| Perfume | 0.15 |

The cream can be prepared according to a conventional method for preparing cosmetic creams.

### Example 4: In vitro assay with human epidermal melanocytes

Normal human epidermal melanocytes were cultured in 48-well plates at a density of 2×10⁴ cells/well overnight in a CO₂ incubator at 37°C in the culture medium called Melanocyte Growth Medium (PromoCell, R.F.A.). The culture medium was then removed and replaced with fresh medium, and the cells were cultured for 96 hours in the presence of a mixture containing 0.0003% (w/w) of hydroxytyrosol, 0.00001% (w/w) of maslinic acid and 0.001% (w/w) of vitamin C. Hydroquinone at a concentration of 10-8% (w/w) was tested as a positive control. To mimic exposure to sunlight, the cells were irradiated with ultraviolet light for the last 48 hours in 4 cycles lasting 15 minutes. Irradiation measurements indicated a total dose of 2.5 J/cm2. Melanin was extracted by adding 1 M sodium hydroxide and incubating at 60°C for 1 h. Melanin was quantified by the absorbance measured at 405 nm, as described, for example, in Kollias et al., Spectroscopic characteristics of human melanin in vivo, J. Invest. Dermatol., 1985, 85(1), 38-42.

In parallel, a cell viability assay was carried out using 3-(4,5-dimethylthiazol-2-yl)-2,5-diphenyltetrazolium bromide dye (MTT Assay) to normalise melanin values to cell levels and thus correct possible interferences due to small variations in the number of cells in each well.

It was observed that melanin production increased by 31% in the control group of melanocytes irradiated with ultraviolet light, with a 49% reduction in the group of melanocytes treated with the ternary combination of the invention and a 42% reduction in the melanocyte group treated with hydroquinone.

Therefore, the ternary combination of the invention was found to reduce the formation of melanin with an efficacy comparable to that of hydroquinone.

### Example 5: In vivo skin treatment trial with the 1% encapsulated ternary combination

The objective of this trial was to evaluate *in vivo* the effects of a dermocosmetic cream, containing 1% (w/w) of the encapsulated ternary combination of Example 2, on skin luminosity, dark spots and skin pigmentation (*Individual Topology Angle,* ITA), after topical application in 20 volunteers for 56 days.

Twenty volunteers, with ages comprised between 40 and 65 years, were enrolled in a 56-day topical treatment program. Each volunteer applied said cream twice a day on the face.

Skin pigmentation (ITA) was measured using a CL 400 Colorimeter^{®} (Courage + Kazaka electronic GmbH), the application of which is described in, for example, Ly *et al., op. cit.,* and the measurements were taken before the first application (D0), and 28 days (D28) and 56 days (D56) after the first application.

Additionally, expert assessments were included in D0, D28 and D56 after the first application to evaluate skin luminosity and dark spots. To determine skin luminosity, a classification scale was defined with photographs of the skin with different degrees of luminosity, where grade 1 corresponded to absence of luminosity, and grade 5 to very shiny skin. Likewise, a classification scale was defined to determine dark spots, which comprised photographs of skin without dark spots (grade 1), up to grade 5, which corresponded to many dark spots. The evaluations were carried out *in vivo* for each of the volunteers.

Lastly, the volunteers filled out a self-assessment questionnaire (usage test) at 28 and 56 days. Dermatological surveillance was included during the study.

The results showed that treatment with a cream, containing the 1% encapsulated ternary combination, increased the ITA value (indicating a decrease in skin pigmentation) by 29.5% ± 5.9% and by 26.1% ± 5.9% after 28 days and 56 days, respectively.

As for the expert evaluation, skin luminosity and the appearance of dark spots were determined after treatment with said cream. The results showed that skin luminosity increased by 48.3% ± 10.12% and 80.8% ± 10.12% after 28 days and 56 days after such treatment, respectively. Moreover, the expert evaluation also determined that this treatment reduced dark spots by 22.8% ± 3.4% after 56 days.

As for skin compatibility and acceptability, none of the volunteers showed any skin acceptability problems or manifested a cutaneous reaction during treatment.

The efficacy of the ternary combination is presented in Table II:

**TABLE II**

| Parameter | Mean value (%)^{a} | Maximum value (%)^{b} |
|---|---|---|
| Colorimeter | | |
| Pigmentation (ITA) (4 weeks) | +30 | +143 |

| Clinical evaluation | | |
|---|---|---|
| Luminosity (8 weeks) | +81 | +300 |
| Reduction of the surface area and number of spots (8 weeks) | -23 | -50 |

| | | |
|---|---|---|
| ^{a}: statistically significant mean variation with respect to the control (D0) ^{b}: maximum variation obtained in a volunteer with respect to the control (D0) | | |

### Example 6: In vivo skin treatment trial with the 2% encapsulated ternary combination

The objective of this trial was to evaluate *in vivo* the effects of a dermocosmetic cream, containing 2% (w/w) of the encapsulated ternary combination of Example 2, on dark spots, skin uniformity, skin pigmentation (ITA) and skin luminosity after topical application in 31 volunteers for 56 days.

Thirty-one volunteers, with ages comprised between 19 and 67 years, were enrolled in a 56-day topical treatment program. Each volunteer applied said cream twice a day on the face.

Skin pigmentation (ITA), the area of dark spots, the number of dark spots, the contrast of dark spots against the skin, and skin uniformity were measured using appropriate equipment, and the measurements were taken before the first application (D0), and 7 days (D7), 14 days (D14), 28 days (D28) and 56 days (D56) after the first application.

For said determinations, a Bio3D scanner combined with a multispectral imaging system was used, as described in Mullor *et al., op. cit.*

Additionally, expert assessments were included in D0, D7, D14, D28 and D56 after the first application to evaluate skin luminosity and dark spots. To determine skin luminosity, a classification scale was defined with photographs of the skin with different degrees of luminosity, where grade 1 corresponded to absence of luminosity, and grade 5 to very shiny skin. Likewise, a classification scale was defined to determine dark spots, which comprised photographs of skin without dark spots (grade 1), up to grade 5, which corresponded to many dark spots. The evaluations were carried out *in vivo* for each of the volunteers.

Lastly, the volunteers filled out a self-assessment questionnaire (usage test) at 7, 14, 28 and 56 days. Dermatological surveillance was included during the study.

The results showed that treatment with a cream, containing the 2% encapsulated ternary combination, increased the ITA value (indicating a decrease in skin pigmentation) by 3.2% ± 1.2%, 2.6% ± 1.2%, 6.0% ± 1.2% and 5.5% ± 1.2% at 7, 14, 28 and 56 days, respectively. Figure 1 shows the image obtained with the Bio3D scanner combined with a multispectral imaging system at the beginning of the trial (D0) and after 56 days (D56), and the reduction in skin pigmentation as a result of treatment with a cream of the invention can be clearly observed.

It was also observed that treatment with the product reduced the area of dark spots by 9.4% ± 2.5%, 11.3% ± 2.5%, 15.2% ± 2.5% and 17.7% ± 2.5% at 7, 14, 28 and 56 days, respectively. Figure 2 shows the image obtained with the Bio3D scanner combined with a multispectral imaging system at the beginning of the trial (D0) and after 56 days (D56), and the reduction of the area of the spots as a result of treatment with a cream of the invention can be clearly observed.

The results also demonstrated that the treatment reduced the number of dark spots by 10.4% ± 3.9% and 14.7% ± 3.9% after 28 and 56 days, respectively.

The effect of treatment with the 2% encapsulation of the invention on the contrast of dark spots against the skin was also determined. In this sense, it was found that this treatment reduced the contrast of dark spots against the skin by 8.3% ± 3.6%, 8.4% ± 3.6% and 11.4% ± 3.6% after 14, 28 and 56 days, respectively. Skin uniformity in response to the treatment, which is a parameter that is proportionally opposite to the contrast of dark spots vs. skin, was calculated, and it was observed that this treatment increased skin uniformity by 8.3% ± 3.6%, 8.4% ± 3.6% and 11.4% ± 3.6% at 14, 28 and 56 days, respectively.

As for the expert evaluation, skin luminosity and the appearance of dark spots were determined after treatment. The results showed that skin luminosity increased by 44.6% ± 5.9%, 68.3% ± 5.9% and 78.0% ± 5.9% after 14, 28 and 56 days of treatment, respectively. Moreover, the expert evaluation also determined that the treatment reduced dark spots by 18.1% ± 2.1%, 24.0% ± 2.1% and 34.5% ± 2.1% after 14, 28 and 56 days of treatment, respectively.

As for skin compatibility and acceptability, none of the volunteers showed any skin acceptability problems or manifested a cutaneous reaction during treatment.

The efficacy of the ternary combination is presented in Table III:

**TABLE III**

| Parameter | Mean value (%)^{a} | Maximum value (%)^{b} |
|---|---|---|
| Bio3D Scanner | | |
| Pigmentation (ITA) (7 days) | +3 | +10 |
| Area of the spots (7 days) | -9 | -24 |
| Area of the spots (8 weeks) | -18 | -73 |
| Number of spots (7 days) | -7 | -67 |
| Number of spots (8 weeks) | -15 | -70 |
| Uniformity (7 days) | +7 | +33 |
| Uniformity (8 weeks) | +11 | +59 |

| Clinical evaluation | | |
|---|---|---|
| Luminosity (7 days) | +26 | +100 |
| Luminosity (4 weeks) | +68 | +200 |
| Reduction of surface area and number of spots (7 days) | -8 | -17 |
| Reduction of the surface area and number of spots (8 weeks) | -35 | -50 |

| | | |
|---|---|---|
| ^{a}: statistically significant mean variation with respect to the control (D0) ^{b}: maximum variation obtained in a volunteer with respect to the control (D0) | | |

The results corresponding to the subgroup of Asian volunteers (5 people) are summarised in Table IV:

**TABLE IV**

| Parameter (8 weeks) | Mean value (%)^{a} | Maximum value (%)^{b} |
|---|---|---|
| Bio3D Scanner | | |
| Pigmentation (ITA) | +8 | +16 |
| Area of the spots | -28 | -73 |
| Number of spots | -33 | -70 |
| Uniformity | +22 | +36 |

| Clinical evaluation | | |
|---|---|---|
| Brightness | +87 | +200 |
| Reduction of surface area and number of spots | -37 | -50 |

| | | |
|---|---|---|
| ^{a}: statistically significant mean variation with respect to the control (D0) ^{b}: maximum variation obtained in a volunteer with respect to the control (D0) | | |

### Example 7: Skin penetration test of the encapsulated ternary combination

The objective of this test was to determine the skin penetration capacity of the nanocapsules containing the ternary combination of the invention.

Three batches of nanocapsules obtained according to the method of Example 1 were tested, but without the presence of the active ingredients of the ternary combination, which had the characteristics of Table V:

**TABLE V**

| Batch of nanocapsules | Mean size D50 ± o.d. | Polydispersity index |
|---|---|---|
| 1 | 531.8±93.59 | 0.543 |
| 2 | 568.10±71.97 | 0.502 |
| 3 | 531.10±67.45 | 0.528 |

0.6 mg of each batch of nanocapsules dispersed in 200 µl of PBS, which contained indocyanine green as a dye, together with a control which was only PBS, were applied to four areas of skin. After 24 hours at 37°C, paraffin cryosections of the treated skin were obtained and analysed with a Leica fluorescence microscope to determine the penetration of the nanocapsules in the skin.

It was observed that all the batches of nanocapsules penetrated the skin, reaching the basal layers of the epidermis, and thereby reaching the target cells. It was also observed that most of the nanocapsules passed through the stratum corneum. Lastly, it was found that these nanocapsules did not induce cytotoxicity in the skin.

## Claims

1. A ternary anti-spot combination to lighten human skin, **characterised in that** it comprises:
a) hydroxytyrosol,
b) maslinic acid and
c) vitamin C.

2. The ternary combination according to claim 1, **characterised in that** hydroxytyrosol is in the form of an *Olea europaea* extract.

3. The ternary combination according to claim 2, **characterised in that** maslinic acid is in the form of an *Olea europaea* extract.

4. The ternary combination according to any of claims 1 to 3, **characterised in that** hydroxytyrosol is in a percentage between 20% (w/w) and 60% (w/w), preferably between 25% (w/w) and 55% (w/w), more preferably between 30% (w/w) and 50% (w/w), and even more preferably between 39% (w/w) and 43% (w/w); maslinic acid is in a percentage between 12% (w/w) and 45% (w/w), preferably between 18% (w/w) and 40% (w/w), more preferably between 20% (w/w) and 34% (w/w), and even more preferably between 25% (w/w) and 29% (w/w); and vitamin C is in a percentage between 15% (w/w) and 50% (w/w), preferably between 20% (w/w) and 45% (w/w), more preferably between 25% (w/w) and 40% (w/w), and even more preferably between 30% (w/w) and 35% (w/w), expressed as ascorbic acid equivalent; wherein all the percentages with respect to the total weight of the ternary combination and the sum of the percentages of the three components is 100%.

5. Nanocapsules, **characterised in that** they comprise:
a) the ternary anti-spot combination to lighten human skin of any of claims 1 to 4,
b) a biodegradable polymer, and
c) at least one cosmetically acceptable ingredient.

6. The nanocapsules according to claim 5, **characterised in that** the biodegradable polymer is selected from polylactic acid (PLA), polyglycolic acid (PGA), poly(lactic-co-glycolic acid) (PLGA), albumin, polycaprolactone (PCL), polybutyrate adipate terephthalate (PBAT), polyesteramides (PEA), polyhydroxyvalerate (PHV), polyalkylene alkanoates (PBS), and thermoplastic starch (TPS); preferably from polylactic acid (PLA), polyglycolic acid (PGL), poly(lactic-co-glycolic acid) (PLGA), albumin, polycaprolactone (PCL), and polyesteramides (PEA); more preferably from polylactic acid (PLA), polyglycolic acid (PGL), poly(lactic-co-glycolic acid) (PLGA), and polycaprolactone (PCL); and even more preferably the biodegradable polymer is polycaprolactone.

7. The nanocapsules according to claim 5 or 6, **characterised in that** the cosmetically acceptable ingredient is selected from surfactants, emulsifiers, lipid compounds, emollients, consistency factors, thickeners, stabilisers, hydrotropes, preservatives, antioxidants, essences, colourants, silicone compounds, fats, waxes, lecithins, phospholipids, film-forming agents, and mixtures thereof; preferably selected from surfactants, emulsifiers, lipid compounds, film-forming agents, and mixtures thereof.

8. A dermocosmetic composition, **characterised in that** it comprises:
a) nanocapsules according to any of claims 5 to 7, and
b) a cosmetically acceptable component.

9. The dermocosmetic composition according to claim 8, **characterised in that** it contains at least one cosmetically acceptable component selected from a cosmetically acceptable carrier, an additional cosmetic active component, an additional cosmetic ingredient and mixtures thereof.

10. The dermocosmetic composition according to claim 9, **characterised in that** the carrier is selected from water, a non-aqueous carrier miscible in water, and a non-aqueous carrier not miscible in water; preferably, the carrier is water.

11. The dermocosmetic composition according to claim 9, **characterised in that** the additional cosmetic active ingredient is selected from skin soothing and healing agents, skin anti-aging agents, skin moisturising agents, anti-wrinkle agents, anti-fibrinolytic agents, anti-atrophy agents, skin softening agents, antibacterial agents, antifungal agents, antimicrobial agents, anti-inflammatory agents, antipruritic agents, external anesthetic agents, keratolytic agents, free radical scavengers, antiseborrheic agents, anti-dandruff agents, agents that modulate differentiation, proliferation or pigmentation of the skin and agents that accelerate penetration, agents that stimulate the synthesis of dermal or epidermal macromolecules and/or prevent their degradation, agents that stimulate the proliferation of fibroblasts and/or keratinocytes or stimulate the differentiation of keratinocytes, muscle relaxers, anti-pollution and/or anti-radical agents, slimming agents, anti-cellulite agents, agents that act on microcirculation, agents that act on the energy metabolism of cells, sunblocks and/or sunscreen compounds, makeup agents, antiseptic agents, deodorant active ingredients, cosmetic astringents, anti-acne agents, anti-caking agents, enzymes, enzyme inhibitors, enzyme inducing agents, coenzymes, plant extracts, plant tissue extracts, plant seed extracts, vitamins, vegetable oils, ceramides, peptides, cosmetic biocides, denaturants, astringent compounds, film formers, vitamins and derivatives thereof, exfoliating agents, lignans, UV absorbents, water soluble sunscreen, antiperspirant, antioxidants, hair remover, fat soluble sunscreen, skin restructuring agent, antiallergics, anti-irritants, immune system stimulants, immune system suppressants, insect repellents, lubricants, photostabilisers, and mixtures thereof.

12. The dermocosmetic composition according to claim 9, **characterised in that** the additional cosmetic ingredient is selected from surfactants, humectants, emulsifiers, lipid compounds, emollients, consistency factors, thickeners, stabilisers, hydrotropes, cosolvents, sequestrants, buffers, preservatives, perfumes, colourants, silicones, fats, waxes, lecithins, phospholipids, UV sunscreen factors, film-forming agents, self-tanners, firmers, pigments, colourants, acids and bases to adjust pH, and mixtures thereof; preferably it is selected from the group consisting of surfactants, humectants, cosolvents, emulsifiers, lipids, emollient compounds, consistency factors, thickening agents, silicones, perfumes, hydrotropes, preservatives, and mixtures thereof.

13. Use of the ternary combination according to any of claims 1 to 4, and of the nanocapsules according to any of claims 5 to 7 to prepare a dermocosmetic composition to lighten human skin.

14. The use according to claim 13, **characterised in that** the dermocosmetic composition is used to reduce the formation of melanin and/or the oxidation thereof.

15. The use according to claim 13, **characterised in that** the dermocosmetic composition is used to reduce the area of skin spots, reduce the contrast of dark spots against the skin, reduce skin pigmentation, the number of skin spots, increase skin luminosity, or increase skin uniformity.
